# EUROPEAN PATENT SPECIFICATION

(11) **EP 3 579 818 B1**
(45) Date of publication and mention of the grant of the patent: **13.10.2021**
(21) Application number: 18701761.1
(22) Date of filing: 30.01.2018
(51) Int. Cl.: A61K 8/41, A61K 8/49, A61Q 5/00, A61K 8/34, A61K 8/42

(54) **A HAIR CONDITIONING COMPOSITION**
HAARPFLEGEZUSAMMENSETZUNG
COMPOSITION DE CONDITIONNEMENT DES CHEVEUX

(30) Priority: 09.02.2017 WO PCT/CN2017/073177; 18.04.2017 EP 17166779
(43) Date of publication of application: 18.12.2019
(73) Proprietor: Unilever Global IP Limited, Wirral, CH62 AZD (GB); Unilever IP Holdings B.V., 3013 AL Rotterdam (NL)
(72) Inventor: CAO, Qunhua, Shanghai, Changning District, 200335 (CN); SUBRAMANIAN, Raghupathi, Shanghai, Changning District 200335 (CN); WU, Xuemeng, Shanghai, Changning District 200335 (CN)
(74) Representative: Tansley, Sally Elizabeth
(86) International application number: PCT/EP2018/052188
(87) International publication number: WO 2018/145941

(56) References cited:
- WO-A1-2010/149424
- WO-A1-2015/085376
- WO-A2-2008/003677
- JP-A- 2011 032 212
- DATABASE GNPD [Online] MINTEL; November 2014 (2014-11), L`Oreal: "Anti-Dandruff Shampoo", XP002771209, Database accession no. 2827449
- Garnier: "Garnier Fructis Mint Detox Anti-Dandruff Sampon", , 2014, XP002771210, Retrieved from the Internet: URL:http://kremmania.hu/kremek/garnier-fru ctis-mint-detox-anti-dandruff-sampon [retrieved on 2017-06-19]

## Description

### Field of the invention

The invention relates to a hair conditioning composition. The invention particularly relates to a more stable and more transparent hair conditioning composition which exhibits anti-dandruff efficacy while delivering the desired sensorial experience in the absence of silicone compounds traditionally included to deliver such benefits.

### Background of the invention

Hair care compositions generally provide cleansing or conditioning benefits or a combination of the two. Hair cleansing compositions generally sold as shampoos typically comprise one or more anionic cleansing surfactants which generally aid in cleaning the hair and the scalp free of undesirable soil, particles and fatty matter. Hair conditioners are another class of hair care compositions which are generally used on hair in the wet condition after the hair has been washed with a shampoo. Compositions which provide the dual benefits of shampooing and conditioning are also known.

Hair conditioners generally comprise cationic surfactants. The conditioning benefit is achieved by including one or more conditioning agents in the hair care composition. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. The most popular hair conditioning agents used are silicones.

Additionally, anti-dandruff benefit has been delivered through hair care compositions, both through shampoos and through hair conditioners. Dandruff is an issue that affects many people globally. The condition is manifested by the shedding of clumps of dead skin cells from the scalp. These are white in colour and provide an aesthetically displeasing appearance. A factor that contributes to dandruff are certain members of the *Malassezia* yeasts. To combat these, anti-dandruff products have included certain zinc salts which have anti-fungal activity, for example zinc pyrithione (ZPTO). Such a product has to perform as a hair cleansing shampoo or as a hair conditioner, while also mitigating the ill-effects of dandruff.

Hair conditioning compositions which comprise silicones have been traditionally used to deliver the desired conditioning benefits as well as the desired sensorial benefits of smoothness, softness and shiny appearance. However silicones have, over the years, acquired certain negative perceptions with some consumers. It is also expected that silicones may face some issues in the future with some regulatory authorities. Further, hair conditioning compositions comprising silicones, especially those that contain anti-dandruff actives, have faced instability problems as they tend to gel and cause grittiness in the product and in certain extreme cases also cause choking and operational problems during the manufacturing stage in the blending vessels and on the agitator blades. Therefore, there has been a trend to find alternatives to silicones which would function equally well, if not better (WO2015/085376 discloses a composition with only a few amount or optional presence of silicone oil). However, the propositions suggested or used so far, have not been able to fully overcome the above problems. The present inventors have solved the above problems by developing a wash-off hair care composition comprising surfactants (especially cationic surfactants) in the presence of an antidandruff active (azole fungicide or Octopirox®(Piroctone Olamine)) with the addition of octyldodecanol The scope of the invention is defined by the appended claims.

WO14100970 (L'Oreal) relates to a keratin fibers conditioning composition, comprising: (a) at least one(C10-C30)alkylamido(C1-C8)alkyl(di)(C1-C6)alkylamine and their cosmetic salts and their solvates such as hydrates; (b) at least one dicarboxylic acid containing at least one hydroxyl group, preferably two hydroxyl groups, the said dicarboxylic acid not bearing a cyclic group; (c) at least one high melting point fatty substance having a melting point of 25 degrees centigrade or higher; and (d) at least one aqueous carrier.

JP2011032212 (Mochida Pharm Co Ltd; Picaso Cosmetic Lab Ltd) relates to hair cosmetic composition containing an antifungal agent comprising a blended azole antifungal agents, surfactants and cationic acylated hydrolyzed collagen.

The above publications do not address the problem of delivering excellent sensorial experience to the consumer along with antidandruff efficacy when silicone oil is not used in a hair conditioning composition nor do they suggest a solution thereof.

It is thus an object of the present invention to deliver equivalent or better sensorial experience to the consumer through a hair conditioning composition which includes minimal or no silicone oils therein.

It is another object of the invention to provide for excellent sensorials from a silicone free hair conditioner while ensuring desired antidandruff efficacy.

It is yet another object of the invention to provide for excellent sensorials and antidandruff efficacy from a silicone free hair conditioning composition that exhibits high transparency.

It is yet another object of the present invention to provide for excellent sensorials, antidandruff efficacy, and enhanced transparency from a silicone free hair conditioning composition while being highly stable and is free of grittiness and is smooth flowing.

### Summary of the invention

The present invention relates to a hair conditioning composition comprising:
a) A cationic surfactant;
b) An antidandruff agent selected from climbazole, ketaconazole or Octopirox®(Piroctone olamine); and
c) from 0.01 to 5.0% by weight of octyldodecanol.
wherein the composition comprises less than 1% silicone compound, preferably less than 0.1% silicone by weight of the composition.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. In other words, in specifying any ranges of values, any particular upper value can be associated with any particular lower value.

The disclosure of the invention, as found herein, is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By 'a Hair Conditioning Composition" as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or soap bar. Non-limiting examples of such compositions include leave-on hair lotions, creams, and wash-off hair conditioner or a shampoo cum conditioner, shower gels, or toilet bars especially a hair conditioning composition. The composition of the present invention is preferably a wash-off composition.

The present invention relates to a hair conditioning composition comprising a cationic surfactant; an antidandruff active selected from climbazole, ketaconazole or Octopirox® (Piroctone olamine); and from 0.01 to 5.0% by weight of octyldodecanol, wherein the composition comprises less than 1% silicone compound, preferably less than 0.1% silicone by weight of the composition.

The composition of the present invention is targeted to compositions which are substantially free of silicone oils. By the phrase 'substantially free of silicone oils is meant that silicone oil is present in an amount less than 1.0%, preferably less than 0.5%, further more preferably less than 0.1% and optimally absent from the composition.

The silicone oil as per this invention is a silicone compound which is a non-volatile silicone. By non-volatile silicone is meant that the silicone has a vapor pressure less than 0.1 mm Hg (13.3 Pa), preferably less than 0.02 mm Hg, more preferably less than 0.01 mm Hg at 25 °C at one atmospheric pressure.

Silicone oils as per this invention include polydiorganosiloxanes, in particular polydimethylsiloxanes which have the CTFA designation dimethicone. Also included within this definition are polydimethyl siloxanes having hydroxyl end groups, which have the CTFA designation dimethiconol. Also included within the definition of silicone oils as per this invention are silicone gums having a slight degree of cross-linking, as are described for example in WO 96/31188 (UNILEVER).

Another class of silicones which is substantially excluded from the hair conditioning compositions of the present invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

The hair conditioning composition of the present invention comprises conditioning surfactants selected from cationic surfactants, used singly or in admixture. Preferably, the cationic surfactants have the formula N⁺R¹R²R³R⁴ wherein R¹, R², R³ and R⁴ are independently (C₁ to C₃₀) alkyl or benzyl. Preferably, one, two or three of R¹, R², R³ and R⁴ are independently (C₄ to C₃₀) alkyl and the other R¹, R², R³ and R⁴ group or groups are (C₁-C₆) alkyl or benzyl. More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine.

The most preferred cationic surfactants for use in the composition are stearamidopropyl dimethylamine, behentrimonium chloride, stearyl trimethyl ammonium chloride or mixtures thereof. In conditioners of the invention, the level of cationic surfactant will generally range from 0.01 % to 5%, preferably 0.1 to 5.0%, more preferably 0.5 to 2.5% by weight of the composition.

Compositions of the invention comprise an anti-dandruff agent selected from Octopirox® (Piroctone olamine), climbazole and ketoconazole. Preferably, the anti-dandruff agent is in solution in the composition. The anti-dandruff agent is therefore preferably soluble in the composition of the invention at 25 °C. Most preferably, the anti-dandruff agent is climbazole (1-imidazolyl-l- (4-chlorophenoxy) -3, 3-dimethylbutan-2-one). The anti-dandruff agent may be a single anti-dandruff compound or a mixture of different anti-dandruff compounds. Preferably, the anti-dandruff agent is present in the composition in an amount of from 0.01 to 5% by weight, more preferably from 0.1 to 5% by weight, most preferably 0.1 to 2% by weight of the composition.

The hair conditioning composition of the invention comprises octyldodecanol. Octyldodecanol is a clear slightly yellow oily material. This is available under the brand name of Eutanol G from Cognis. It is included in an amount of 0.01 to 5.0%, preferably 0.1 to 3% by weight of the composition.

Without wishing to be bound by theory, the present inventors believe that the benefits of the invention in terms of less grittiness and more transparency is achieved as a result of minimizing agglomeration of particles. The inventors believe that the rate of agglomeration of particles that are formed as a consequence of using conventional ingredients like zinc pyrithione and silicone oil especially in the presence of certain perfumery compounds is quite high leading to emulsion breakdown. This has been minimized by utilizing azole compound in combination with octyldodecanol.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

According to another aspect of the present invention there is provided a process to prepare a stable and more transparent hair conditioning composition of the invention comprising the step of mixing the ingredients of the compositions.

The invention will now be described with reference to the following non-limiting examples.

### Examples

### Examples 1 -3: Grittiness and transparency exhibited by various conditioner compositions

The following hair conditioning compositions as shown in Table - 1 were prepared. The compositions were tested for stability using the following procedure.

The stability was measured by determining the amount of gritty matter that is formed in the composition that gels and sticks to the vessel, the agitator and the sieve. The %grittiness was measured using the following procedure.

Test procedure: About 800g of the conditioner is taken in a 1000ml beaker and stirred with a flat paddle at 160 rpm for 20 minutes. Then about 700g of the conditioner sample is taken out of the beaker, leaving about 100 g of the conditioner in the beaker. About 700 g of hot water (80-90 °C) is poured into the beaker and stirred with a flat paddle at 160 rpm for 20 minutes. The diluted mass is then filtered using a 710 µm mesh. The contents of the beaker are then filled with 800 ml water (25 °C) and stirred at 160 rpm for 1 minute. The beaker, the mesh and the paddle are then dried at 45 °C for 3 hours. The increase in weight of the beaker, the paddle and the mesh is then measured which is an indication of the absolute amount of grit. The % grittiness is then calculated using the equation: % grittiness = (absolute amount of grit) / (amount of the hair conditioner taken, 100g) * 100.

The % grittiness as measured for the various samples are summarized in Table -1 below.

**Table - 1**

| Components, wt% | Ex1 | Ex2 | Ex3 |
|---|---|---|---|
| Behentrimonium chloride | 0.7 | 0.7 | 0.7 |
| Stearamidoproyl dimethyl amine | 1 | 1 | 1 |
| Cetearyl alcohol | 4 | 4 | 4 |
| Lactic Acid | 0.304 | 0.304 | 0.304 |
| Zinc pyrithione | 0.50 | - | - |
| Climbazole | - | 0.48 | 0.48 |
| Silicone oil | 5.0 | 5.0 | - |
| Octyldodecanol | - | - | 1.5 |
| Water | To 100 | To 100 | To 100 |
| % grittiness | 0.83 | 0.12 | 0.03 |

The data in table -1 above indicates that the combination of climbazole with octyldodecanol (Example -3) is vastly superior to that when zinc pyrithione is used in combination with silicone oil (Example -1) or when climbazole is used in combination with silicone oil (Example -2).

### Transparency as measured using transmittance

Samples of Example 1 and Example 3 were tested for their transparency by measuring the % transmittance at various wavelengths. The procedure used to measure the transmittance is as given below:

The %Transmittance value is the %ratio of the radiant flux transmitted through and emerging from a body to the total flux incident on it. The Equipment used was Ultra Scan Pro HunterLab Spectrophotometer in Total Transmittance Mode (TTRAN) mode with a transmission cell of 10 mm width. In this method, the sample is placed on the port of the integrating sphere to effectively collect all diffusely and non-diffusely transmitted light. The % transmittance values of the samples at various wavelengths is summarized in Table - 2 below:

**Table - 2**

| | % Transmittance | | | |
|---|---|---|---|---|
| Wavelength | 400 nm | 500 nm | 600 nm | 700 nm |
| Example -1 | 0.5 | 3.6 | 4.7 | 5.2 |
| Example - 3 | 3.0 | 8.2 | 9.6 | 10.4 |

The data in Table - 2 above indicates that a composition as per the invention (Example -3) exhibits higher transparency as compared to a composition comprising conventional ingredients (Example 1 having ZPTO + Silicone oil).

### Sensory testing

The sensorials delivered by the composition of the invention (Example 3) was compared to that of a composition outside the invention (Example 1) in a Salon trial. In the salon trial the samples were tested on 36 female respondents. The respondents were evaluated prior to the trial to have the same type of hair (e.g. normal hair, dry hair, greasy hair etc). In a given trial, 18 respondents with one type of hair and 18 with another type of hair were chosen. The evaluation was done by trained hairdressers with good sensitivity. The evaluation method involved paired comparison with half the head washed with one sample and the other half of the head washed with the other sample. The procedure used was as follows:
Step 1: 4 ml of shampoo and 4 ml of conditioner was weighed.
Step 2: Each of the two samples were used to wash each half of the head by the hairdresser.
Step 3: Wet Stage Evaluation was carried out by both the hairdresser and the consumer.
Step 4: Drying of the hair by the consumer.
Step 5: Dry stage evaluation by both the consumer and the hairdresser.

The following attributes were scored during the various stages of the conditioner use: During application: Visual absorbancy, Ease of detangling, speed of incorporation, slippery feel and product coating
During rinse off: Maximum slippery feel, longer time to rinse strip, easier to finger through during rinse, more slippery feel after rinse (under running water).
Wet stage: Easier wet combing (total effort), Easier wet combing (single stroke), More slippery feel wet.
During dry stage: Easier dry combing (total effort), Easier dry combing (single stroke), volume, more visual expansion from scalp, Less stick out, More shiny, More slippery feel, Hair surface smoother, more elastic when compressed, Higher hair dryness, more coating, finger through, more weighty hair more sticky, and static (easier).

When a salon trial with Example -1 was compared to Example -3, it was found that there is no significant difference between the two samples in all of the above attributes in all of the various stages mentioned above.

The data from the salon trial and that in Table -1 and 2 indicates that the composition as per the invention (Example 3) has comparable sensorials when in use as compared to a conventional composition (Example -1) while exhibiting less grittiness (thereby being more stable) and also exhibits better transparency.

## Claims

1. A hair conditioning composition comprising
a) A cationic surfactant;
b) An antidandruff agent selected from climbazole, ketoconazole, or octopirox; and
c) from 0.01 to 5.0% by weight of octyldodecanol;
wherein the composition comprises less than 1% silicone compound, preferably less than 0.1% silicone compound by weight of the composition.

2. A composition as claimed in claim 1 wherein the antidandruff agent is climbazole.

3. A composition as claimed in claims 1 or 2 wherein the antidandruff agent is present in an amount of 0.01 to 5% by weight of the composition.

4. A composition as claimed in any one of the preceding claims wherein the surfactant is present in an amount of from 0.01 to 5.0% by weight of the composition.

5. A composition as claimed in any one of the preceding claims wherein the cationic surfactant is chosen from stearamidopropyl dimethylamine, behentrimonium chloride, stearyl trimethyl ammonium chloride or mixtures thereof.

6. A composition as claimed in any one of the preceding claims additionally comprising 0.5 to 10 wt% of a fatty alcohol, wherein the fatty alcohol has a carbon chain length of 8 to 22 and the fatty alcohol are compounds containing straight chain alkyl groups.

7. A process to prepare a stable and more transparent hair conditioning composition as claimed in any one of the preceding claims comprising the step of mixing the ingredients of the composition as claimed in claim 1.

## Patentansprüche

1. Haarkonditionierungszusammensetzung, umfassend
a) ein kationisches Tensid;
b) ein Antischuppenmittel, ausgewählt aus Climbazol, Ketoconazol oder Octopirox; und
c) 0,01 bis 5,0 Gewichts-% Octyldodecanol;
wobei die Zusammensetzung weniger als 1% Siliconverbindung, bevorzugt weniger als 0,1% Siliconverbindung, bezogen auf das Gewicht der Zusammensetzung, umfasst.

2. Zusammensetzung wie in Anspruch 1 beansprucht, wobei das Antischuppenmittel Climazol ist.

3. Zusammensetzung wie in den Ansprüchen 1 oder 2 beansprucht, wobei das Antischuppenmittel in einer Menge von 0,01 bis 5 Gewichts-% der Zusammensetzung vorliegt.

4. Zusammensetzung wie in irgendeinem der vorhergehenden Ansprüche beansprucht, wobei das Tensid in einer Menge von 0,01 bis 5,0 Gewichts-% der Zusammensetzung vorliegt.

5. Zusammensetzung wie in irgendeinem der vorhergehenden Ansprüche beansprucht, wobei das kationische Tensid ausgewählt ist aus Stearamidpropyldimethylamin, Behentrimoniumchlorid, Stearyltrimethylammoniumchlorid oder Mischungen davon.

6. Zusammensetzung wie in irgendeinem der vorhergehenden Ansprüche beansprucht, zusätzlich umfassend 0,5 bis 10 Gew.-% eines Fettalkohols, wobei der Fettalkohol eine Kohlenstoffkettenlänge von 8 bis 22 aufweist und der Fettalkohol Verbindungen sind, die geradkettige Alkylgruppen enthalten.

7. Verfahren zur Herstellung einer stabilen und transparenteren Haarkonditionierungszusammensetzung wie in irgendeinem der vorhergehenden Ansprüche beansprucht, umfassend den Schritt des Mischens der Bestandteile der Zusammensetzung wie in Anspruch 1 beansprucht.

## Revendications

1. Composition de conditionnement des cheveux comprenant
a) un tensioactif cationique ;
b) un agent antipelliculaire choisi parmi le climbazole, kétoconazole, ou octopirox ; et
c) de 0,01 à 5,0 % en masse d'octyldodécanol ;
dans laquelle la composition comprend moins de 1 % de composé de silicone, de préférence moins de 0,1 % de composé de silicone en masse de la composition.

2. Composition selon la revendication 1, dans laquelle l'agent antipelliculaire est le climbazole.

3. Composition selon la revendication 1 ou 2, dans laquelle l'agent antipelliculaire est présent dans une quantité de 0,01 à 5 % en masse de la composition.

4. Composition selon l'une quelconque des revendications précédentes, dans laquelle le tensioactif est présent dans une quantité de 0,01 à 5,0 % en masse de la composition.

5. Composition selon l'une quelconque des revendications précédentes, dans laquelle le tensioactif cationique est choisi parmi la stéaramidopropyldiméthylamine, le chlorure de béhentrimonium, le chlorure de stéaryltriméthylammonium ou des mélanges de ceux-ci.

6. Composition selon l'une quelconque des revendications précédentes comprenant de plus de 0,5 à 10 % en masse d'un alcool gras, dans laquelle l'alcool gras présente une longueur de chaîne carbonée de 8 à 22 et les alcools gras sont des composés contenant des groupes alkyle linéaires.

7. Procédé pour préparer une composition de conditionnement des cheveux stable et plus transparente selon l'une quelconque des revendications précédentes comprenant l'étape de mélange des ingrédients de la composition selon la revendication 1.
